# EUROPÄISCHE PATENTSCHRIFT

(11) **EP 2 319 485 B1**
(45) Veröffentlichungstag und Bekanntmachung des Hinweises auf die Patenterteilung: **07.03.2018**
(21) Anmeldenummer: 10178819.8
(22) Anmeldetag: 23.09.2010
(51) Int. Cl.: A61K 8/23, A61K 8/55, A61K 8/898, A61Q 5/12

(54) **Gilbfreie Haarbehandlungsmittel mit Aminosilikonen**
Non-yellowing hair treatment agent with amino silicones
Agent de traitement des cheveux contenant des silicones aminés et ne subissant pas de jaunissement

(30) Priorität: 09.10.2009 DE 102009048978
(43) Veröffentlichungstag der Anmeldung: 11.05.2011
(73) Patentinhaber: Beiersdorf AG, 20253 Hamburg (DE)
(72) Erfinder: Saladin, Sandra, 20144, Hamburg (DE); Ahlheit, Sabrina, 22303, Hamburg (DE); Klauck, Robert, 21465, Reinbek (DE)

(56) Entgegenhaltungen:
- EP-A1- 1 123 374
- EP-A2- 0 198 788
- EP-B1- 1 287 051
- WO-A2-02/078661
- FR-A1- 2 814 475
- JP-A- 2008 143 817

## Beschreibung

Die vorliegende Erfindung betrifft gilbfreie Haarbehandlungsmittel mit Aminosilikonen und insbesondere Haarspülungen.

Haarspülungen werden zur Pflege nach der Reinigung der Haare angewendet. Übliche Haarspülungen werden auf Basis von kationischen Tensiden wie beispielsweise Cetyl Trimethyl Ammonium Chlorid und Fettalkoholen wie Cetyl und/oder Stearylalkohol formuliert. Allein reicht diese Kombination jedoch nicht für eine geeignete konditionierende Pflege an Haaren wie unter anderem Geschmeidigkeit, Kämmbarkeit und Glanz aus. Aus diesem Grund werden häufiger Silikone wie beispielsweise langkettige Polydimethylsiloxane eingesetzt. Noch substantiver sind Silikone, die primäre und sekundäre Aminogruppen in der Seitenkette enthalten. In einer Spülungsformulierung bei pH-Werten von 3 bis 5 sind diese Gruppen protoniert, so dass die kationische Natur des Silikons zu einer erhöhten Konditionierleistung führt. Nachteilig ist abhängig vom Aminosilikon-Typ eine mit der Zeit zunehmende Vergilbung von kosmetischen Formulierungen, die Aminosilikone (bekannt auch als Amodimethicone) enthalten. Die Vergilbung von kosmetischen Formulierungen, die Amodimethicone, besonders Amodimethicone Fluide enthalten, ist eine bekannte Herausforderung in der Kosmetik und in der Textilchemie (Lacasse, K.; Baumann, W. in Textile Chemicals Environmental Data and Facts. Springer Verlag, 2004, S.387.) Ursache ist die Oxidierung von Aminen (CRC-Oxidation Inhibition in Organic Materials, Volume-II). Chelat Liganden wie EDTA (Ethylendiamintetraacetat) oder Antioxidantien wie BHT (Butylierte Hydroxytoluol-Derivate) können den Vergilbungsprozess nur verlangsamen und nicht beseitigen. Hersteller wie z.B. Dow Corning oder Blue Star entwickeln amino funktionalisierte Silikone, wie Dow Corning 8500 Conditioning Agent (Bis(C13-C15 Alkoxy)PG-Amodimethicone oder Mirasil HALS Aminosilikone ADMH 45 und ADMH 50 (INCI: Propoxytetramethylpiperidinyl Dimethicone) um die Vergilbung zu minimieren. Die Vergilbung von Aminen führt dazu, dass möglich vielversprechende aminsubstituierte Silikone entweder nicht zum Einsatz kommen oder nur in geringen Einsatzkonzentrationen verwendet werden können. Häufig werden Marktprodukte eingefärbt (lila, rot, braun, gelb) um die Vergilbung zu kaschieren. Die Verhinderung der Vergilbung von Aminosilikon bietet Vorteile für kosmetische Formulierungen, weil eine größere Bandbreite an aminhaltigen Silikonen verwendet werden kann.
Der Stand der Technik kennt Na-disulfit (Na₂S₂O₅). Es ist gut wasserlöslich und bildet dort Natriumbisulfit NaHSO₃ (aq.). Die Substanz ist im Annex der Kosmetikverordnung als Konservierungsmittel gelistet.

EP-0198788A2 offenbart die Erhöhung der Lagerstabilität von Mischungen aus Aminen und Imidazoliniumverbindungen durch ein Bisulfit.

EP 1 714 634 offenbart ein Haarbehandlungs-Kit, umfassend
- mindestens ein Kompartiment, welches ein Mittel beinhaltet, das mindestens einen Komplexbildner enthält;
- mindestens ein vom ersten Kompartiment verschiedenes Färbekompartiment, welches Mittel zum Färben von keratinhaltigen Fasern, insbesondere menschlichen Haaren, enthält.

EP 1679061B1 offenbart eine Konditionierzubereitung mit einer Haarbehandlungsverbindung und einem Farbstoff, einem Chelator und einem UV-Absorber.

EP 0 622 070 B1 offenbart eine Pulvermischung mit einer Formaldehyd und/oder Ameisensäure freisetzenden Verbindung mit hautbräunenden Eigenschaften und ein Sulfitionen bildendes Agens und gegebenenfalls einen Stabilisator.

EP 0 752 842 offenbart eine künstliche Hautbräunungszubereitung mit Dihydroxyaceton in einem topischen Träger mit Metabisulfit-, Sulfit- oder Hydrogensulfitsalzen.
JP 2008143817 offenbart eine Emulsionszubereitung, die neben Amino-modifizierten Silikonen Hydroxyethandiphosphonsäure und/oder ihre Salze enthält, um der Vergilbung der Zubereitung entgegenzuwirken. Derartige Zubereitungen werden bevorzugt zur Haarreparatur und Haarbehandlung eingesetzt.

Weiterer Stand der Technik findet sich in WO 1998019665, EP 0 198 788A2, EP 1 287 051 B1, US 20070062653, EP 1 289 478, EP 0 859 587 B1, EP 0 617 953 B1, WO 2002102334 A2 und in EP 1 586 298 B1.

All dies konnte die Mängel des Standes der Technik nicht beseitigen. Überraschend und für den Fachmann nicht vorhersehbar hat sich nun herausgestellt, dass eine haarkosmetische Zubereitung enthaltend Aminosilikone, Natriumdisulfit (auch Sodium Metabisulfite genannt) und Etidronsäure und oder deren Natrium- oder Kaliumsalz den Mängeln des Standes der Technik abhilft. Die Kombination von dem Reduktionsmittel Natriumdisulfit (Na₂S₂O₅) mit dem Chelat-Liganden Etidronsäure in einer Haarpflegeformel, besonders in einer Haarspülung, verhindert die Vergilbung von Aminosilikonen über einen Beobachtungszeitraum von 120 Tagen bei Raumtemperatur und 40°C im Brutschrank. Im Wechseltemperaturschrank (-10°C bis 40°C im 12 Std.-Wechsel), Kühlschrank bei +6°C und im Eisschrank bei -10°C wurde ebenfalls standardgemäß 28 Tage geprüft und es kam zu keiner Verfärbung. Mit dem Einsatz von Etidronsäure findet eine Vergilbung bereits nach 14 Tagen statt und mit Natrium-disulfit wurde bei 40°C im Brutschrank eine anfängliche Vergilbung ab 30 Tagen festgestellt. Natrium-disulfit wird mit dem Handelsnamen Natriumdisulfit food grade (E223) von Biesterfeld angeboten (Hersteller BASF) und ist für diese Erfindung bevorzugt. Weitere geeignete Sulfite sind die Akali-Metall-Sulfite (Li, Na, K). Eine gute Referenz für Metabisulfit ist The Merck Index, Tenth Edition (1983), Sodium Metabisulfite, Entry 8476, p.1237, Sodium Suflite, Entry 8528, and Sodium Bisulfite, Entry 8419, p.1231. Etidronsäure ist unter dem Handelsnamen Turpinal SL von Henkel, Thermphos Trading bekannt. Weitere erfindungsgemäße Chelat-Liganden sind Tetrasodium Etidronate (Turpinal 4 NL, Henkel) sowie auch Tetrapotassium Etidronate (Wayhib-RW, Arch Chemicals). Weiterhin hat sich überraschenderweise gezeigt, dass Spülungen mit Etidronsäure und Na-disulfit sich durch eine verbesserte Ausspülbarkeit auszeichneten. Es ist vom Verbraucher erwünscht ein gepflegtes Haargefühl ohne Beschwerung der Haare zu haben, die durch eine optimale Ausspülbarkeit des Haarpflegeprodukts zu gewährleisten ist.

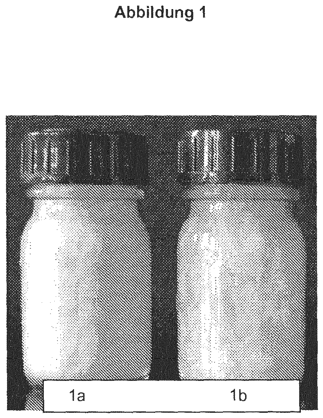

Kationische Amine, insbesondere tertiäre Amine lassen sich in Kombination mit Fettalkoholen wie Cetyl und Stearyl Alkohol zu einer reichhaltigen, cremigen Spülungsgrundlage formulieren. Solche Basis-Spülungsformeln wurden mit Amidominen formuliert und verschiedenste Aminosilikone wurden dazugesetzt. Gemäß dieser Erfindung erwies sich die Kombination von zwei Aminosilikonfluiden - Dow Corning AP-8087 und Dow Corning 2-8566 als besonders vorteilhaft bez. Kämmbarkeit und Pflegeeigenschaften an 2Std. gebleichten 2g Haarsträhnen. Jedoch zeigte diese Formulierung eine starke Vergilbung besonders nach 180 Tagen bei Raumtemperatur und 40°C im Brutschrank, wie auf Abbildung 1 zu sehen ist.

Alle Angaben in % in diesem Dokument sind Gew.%, sofern nicht anders angegeben.

Abbildung 1 zeigt eine Spülung mit Aminosilikonen 1.0 % AP-8087 & 0.5% DC 2-8566 gemäß Formel 1(Tabelle 1).
*(Spülungen sind in Weithalsflaschen, 30mL, Klarglas, Gewinde: GL32, Mit Verschluss 1* - *Hersteller Rixius gelagert)*
1a: RT - 180 Tage Lagerung bei 25°C; **L*:** 57.0, **a***:-2.1, **b***: 2.6
1b: B40 - 180 Tage Lagerung im Brutschrank bei 40°C; **L*:** 56.6, **a***:-2.6, **b***: 4.1
Der Parameter "Verfärbung" wurde sowohl photographisch sowie auch mittels eine handelsübliche Methode, zur sicheren Messung von Farben aufgenommen - Dr. LANGE SPECTRO-PEN (Gerätenummer 1013141) http://www.gardco.com/pages/color/spectro_color.html. Das SPECTRO-PEN ist ein portables Farbmessgerät mit Kugelgeometrie.

Der L*a*b*-Farbraum ist ein Messraum in dem alle wahrnehmbaren Farben enthalten sind (Beuth-Verlag: DIN 6174: Farbmetrische Bestimmung von Farbmaßzahlen und Farbabständen im angenähert gleichförmigen CIELAB-Farbenraum (Ausgabe DIN 6174:2007-10; abgerufen am 15. Juli 2009)). Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Der L*a*b*-Farbraum wird durch ein dreidimensionales Koordinatensystem beschrieben. Die a*-Achse beschreibt den Grün- oder Rotanteil einer Farbe, wobei negative Werte für Grün und positive Werte für Rot stehen. Die b*-Achse beschreibt den Blau- oder Gelbanteil einer Farbe, wobei negative Werte für Blau und positive Werte für Gelb stehen. Die Skalen der a*-Achse und der b*-Achse umfassen einen Zahlenbereich von -150 bis +100 und -100 bis +150, ungeachtet dessen, dass es für einige Werte keine wahrnehmbare Entsprechung gibt. Da die Farbwhrnehmung bei der Erstellung dieses Farbraumes berücksichtigt wurde, ist der daraus resultierende Farbkörper bei kartesischen Koordinaten ungleichförmig.

Die L*-Achse beschreibt die Helligkeit (Luminanz) der Farbe mit Werten von 0 bis 100.

Im Vergleich zum ursprünglichen XYZ-Raum der CIE-Normfarbtafel ist im a*-b*-System der Grünbereich kleiner, während der Purpur-Blau-Cyanbereich größer ist.

Einen ungefähren Eindruck davon soll die Kurvenschar im a*-b*-Diagramm geben. Während Gelb und Grün Werte bis zu 150 erreichen können, belegen Rot und Blau Zahlenwerte nur bis 100.

Verschiedene Chelatliganden haben zu keiner Verminderung der Vergilbung geführt. Verschiedene Antioxidantien haben ebenfalls keine Verbesserung gebracht.

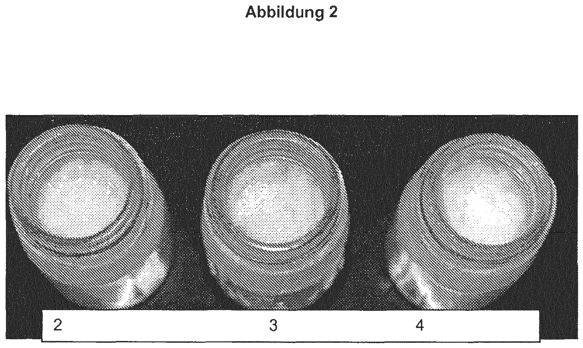

Der Einsatz vom Reduktionsmittel Na-Disulfit (0.15%)aktiv führte zu einer deutlichen Verbesserung wie in Abbildung 2 zu sehen ist. Zusatz von 0.18% aktiv Etidronsäure C₂H₈O₇P₂ (Turpinal SL, Thermphos Trading) zu der Formel enthaltend Na-Disulfit (0.15%) führte zu keiner Vergilbung der Formel im Beobachtungszeitraum von 120 Tagen. Die Etidronsäure allein war nicht ausreichend für die Verhinderung der Vergilbung (siehe Abbildung 2).

Abbildung 2 zeigt Spülungen mit Aminosilikonen AP-8087 & DC 2-8566 gemäß Formel 1 (Tabelle 1) bei 40°C Lagerung im Brutschrank 120 Tage. *(Spülungen sind in Weithalsflaschen, 30mL, Klarglas, Gewinde: GL32, Mit Verschluss 1* - *Hersteller Rixius gelagert)*
2: 0.15% Na₂S₂O₆ **L*:** 60.7, **a***:-2.8, **b***: 0.8
3: 0.18% C₂H₈O₇P₂ **L*:** 56.6, **a***:-2.4, **b***: 3.2
4: 0.18% C₂HₛO₇P₂ + 0.15% Na₂S₂O₆ **L*:** 56.9, **a***:-3.3, **b***: 0.5

| | inci | **1 [Gew.%]** | **2 [Gew.%]** | **3 [Gew.%]** | **4 [Gew.%]** |
|---|---|---|---|---|---|
| 1 | Methylparaben | 0,25 | 0,25 | 0,25 | 0,25 |
| 2 | Propylparaben | 0,10 | 0,10 | 0,10 | 0,10 |
| 3 | Aqua | Ad.100 | Ad.100 | Ad.100 | Ad.100 |
| 4 | Cetyl Alcohol | 2,20 | 2,20 | 2,20 | 2,20 |
| 5 | Lactic Acid | 0,84 | 0,80 | 0,76 | 0,76 |
| 6 | Sodium Chloride | 0,01 | 0,01 | 0,01 | 0,01 |
| 7 | Stearyl Alcohol | 4,40 | 4,40 | 4,40 | 4,40 |
| 8 | Etidronic Acid | | | 0,30 | 0,30 |
| 9 | Oryzanol | 0,05 | 0,05 | 0,05 | 0,05 |
| 10 | Coco Betaine | 0,85 | 0,85 | 0,85 | 0,85 |
| 11 | Stearamidopropyl Dimethylamine | 2,20 | 2,20 | 2,20 | 2,20 |
| 12 | Amodimethicone | 0,50 | 0,50 | 0,50 | 0,50 |
| 13 | Sodium Metabisulfite | | 0,15 | | 0,15 |
| 14 | Bis-Hydroxy/Methoxy Amodimethicone | 1,00 | 1,00 | 1,00 | 1,00 |
| 15 | Perfume | 0,70 | 0,70 | 0,70 | 0,70 |

| | **Tabelle 1** | |
|---|---|---|
| | **Handelsnamen der Rohstoffe (% aktiv Hauptkomponente):** | |
| 1 | Nipagin M, Clariant, 99.5% | |
| 2 | Nipasol M, Clariant, 99.5% | |
| 4 | Nacol 16-95, Sasol , 95% | |
| 5 | 100366 Milchsäure 90, reinst DAB, Merck, 90% | |
| 7 | Lanette 18, Cognis , 100% | |
| 8 | Turpinal SL, Henkel or, Thermphos Trading, 60% | |
| 9 | Gamma -Oryzanol, Biesterfeld, 98% | |
| 10 | Dehyton AB 30, Cognis, 31% | |
| 11 | Tego Amid S18, Evonik Goldschmidt, 100% | |
| 12 | Dow Corning 2-8566, 100% | |
| 13 | Natriumdisulfit food grade (E223), Biesterfeld | |
| 14 | Dow Corning AP-8087, 100% | |

Die b*-Achse beschreibt den Blau- oder Gelbanteil einer Farbe, wobei negative Werte für Blau und positive Werte für Gelb stehen, so dass höhere Wert für b* eine Erhöhung der Vergilbung bedeutet.

| | inci | **1** | **4** |
|---|---|---|---|
| 1 | Methylparaben | 0,25 | 0,25 |
| 2 | Propylparaben | 0,10 | 0,10 |
| 3 | Aqua . | Ad.100 | Ad.100 |
| 4 | Cetyl Alcohol | 2,20 | 2,20 |
| 5 | Lactic Acid | 0,84 | 0,76 |
| 6 | Sodium Chloride | 0,01 | 0,01 |
| 7 | Stearyl Alcohol | 4,40 | 4,40 |
| 8 | Etidronic Acid | | 0,30 |
| 9 | Oryzanol | 0,05 | 0,05 |
| 10 | Coco Betaine | 0,85 | 0,85 |
| 11 | Stearamidopropyl Dimethylamine | 2,20 | 2,20 |
| 12 | Amodimethicone | 0,50 | 0,50 |
| 13 | Sodium Metabisulfite | | 0,15 |
| 14 | Bis-Hydroxy/Methoxy Amodimethicone | 1,00 | 1,00 |
| 15 | Perfume | 0,70 | 0,70 |
| | Ausspülbarkeit (Zeit, Sekunden, Mittelwert) | 22 | 14 |

**Tabelle 3.**

| | **Handelsnamen der Silikone (% aktiv Hauptkomponente):** |
|---|---|
| 12 | Dow Corning 2-8566, 100% |
| 14 | Dow Corning AP-8087, 100% |

Haarspülungen werden zur Pflege nach der Reinigung der Haare angewendet. Ein wichtiger Faktor ist die Sensorik der Spülung während des Auftragens auf das Haar sowie auch die Ausspülbarkeit des Produktes. Eine Spülung sollte zu einem angenehmen Haargefühl im nassen Haar führen, jedoch das Haar nicht während des Ausspülens beschweren. Der Verbraucher sollte das Produkt nicht mehr auf dem Haar spüren, jedoch sollte das Haar gepflegt und weich sein. Die "Ausspülbarkeit" eines Produkts wird im Sinne dieser Schrift wie folgt festgelegt: Mit dem Daumen und Zeige/Mittelfinger einer Hand wird erfühlt beim Ausspülen, wie leicht sich das Produkt aus den Haaren der Strähne auswaschen lässt. Die Zeit (in Sekunden) die vergeht, bis kein Produkt mehr zu fühlen ist, wird notiert. Von einem Expertenteam wurden zunächst zwei haarkonditionierende Formeln (je 0,4mL Produkt wird auf zwei 2std. gebleichten, 2g Haarsträhnen von der Firma Kerling aufgetragen, 1min lang massiert, 30Sekunden lang einwirken gelassen und danach ausgespült. Die Ausspülzeit wird notiert.) mit und ohne (aktiv: 0.18% C₂H₈O₇P₂ + 0.15% Na₂S₂O₅) evaluiert (Tabelle 3). Bei der sensorischen Bewertung wird eine 10er Skala verwendet bei der 10 den höchsten Wert darstellt. Formel 4 wurde 8 Sekunden schneller ausgespült als Formel 1 bei nahezu gleichbleibenden Haarpflegeeigenschaften (Gleitvermögen, Haarstruktur während der ganzen Anwendung, Nasskämmbarkeit) bis auf die Produkt Reichhaltigkeit, die um einen Punkt abfällt.

Bevorzugt ist es, wenn die Zubereitung höchstens 0,1% Farbstoffe enthält. Weiter bevorzugt ist es, wenn ein kationisches Tensid enthalten ist. Bevorzugt ist in Spülungen besonders Stearamidopropyl Dimethylamine als kationischer Pflegestoff. Eine besonders bevorzugte Spülungsformel enthält als ein weiteres Tensid zusätzlich Coco-Betaine in einem Gehalt von 0.85 Gew.%.

Weiter bevorzugt ist es, wenn eine oder mehrere Fettverbindungen mit einem Schmelzpunkt oberhalb von 40°C enthalten sind. Weiter bevorzugt ist es, wenn das Aminosilikon 0.5 - 2.5 mol% Amine enthält. Weiter bevorzugt ist es, wenn die Zubereitung eine Haarspülung ist. Weiter bevorzugt ist es, wenn zwei verschiedene Aminosilikone enthalten sind. Weiter bevorzugt ist es, wenn das zweite Aminosilikon als Fluid mit einer Viskosität von 1000cst bis 6000cst, vorzugsweise von 1000cst bis 5000cst vorliegt. Geeignet sind aber auch Aminosilkone mit Viskositäten höher als 10,000cs. Aminosilikone können als Fluid und Emulsion vorliegen, aber besonders bevorzugt als Fluide. Besonders bevorzugt ist es, wenn das zweite Aminosilikon als Microemulsion von einem kationischen amino-functionalisertien Silikonpolymer mit einer Viskosität von 10,000cs vorliegt. Weiter bevorzugt ist es, wenn als Aminosilikone Amino-funktionalisiertes Bis-Hydroxy/Methoxy Amodimethicone der Formel 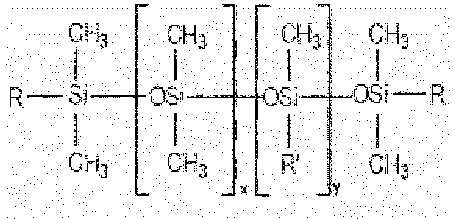 where R represents -OH or -OCH₃ and R' is 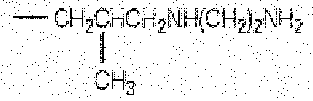 mit einer Viskosität von 5000cP und einem Amingehalt von 0.5mol% diamine - Dow Corning AP-8087 oder Amodimethicone, dies ist ein aminofunktionalisiertes Polydimethylsiloxane mit einem hohen Amingehalt (2.3% mole Amin), das als Fluid vorliegt und eine Viscosität von 3500cSt aufweist, eingesetzt werden.

Amodimethicone wird durch folgende Struktur gekennzeichnet:

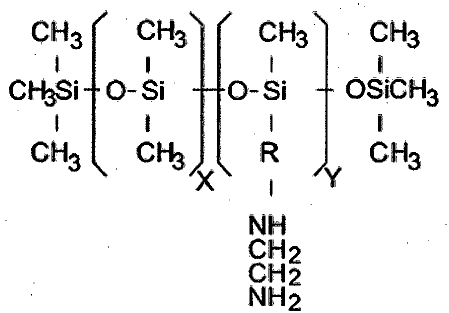

Weiter bevorzugt ist es, wenn 0.10 bis 0.5 Gew.% besonders bevorzugt 0.15 bis 0.2 Gew.% Na-disulfit jeweils bezogen auf den Aktivgehalt enthalten sind. Weiter bevorzugt ist es, wenn der Gehalt an Etidronsäure wenigstens dem Gehalt an Na-disulfit entspricht. Weiter bevorzugt ist es, wenn der Gehalt an EDTA höchstens 0,01 Gew.% beträgt. Weiter bevorzugt ist es, wenn der Gehalt an pflanzlichen Ölen gewählt aus der Gruppe Sonnenblumenöl, Babassuöl, Jojobaöl, Avocadoöl höchstens 1 Gew.% beträgt. Weiter bevorzugt ist es, wenn der Gehalt an pflanzlichen Extrakten gewählt aus der Gruppe hydrolysierte Seide, Aloe, Kamille, lily of the valley höchstens 1 Gew.% beträgt. Weiter bevorzugt ist es, wenn der Gehalt an pflanzlichen Ölen und an pflanzlichen Extrakten zusammengenommen höchstens 1 Gew.% beträgt. Weiter bevorzugt ist es, wenn der Gehalt an pflanzlichen Ölen höchstens 1 Gew.% beträgt. Die Erfindung umfasst auch die Verwendung von Natriumdisulfit und Etidronsäure zur Verhinderung des Vergilbens haarkosmetischer Zubereitungen mit Aminosilikonen.

Bevorzugt ist es, wenn folgende Aminosilikonfluide eingesetzt werden:
*angeboten von der Gesellschaft Dow Corning:*
   Fluide: 2-8566, AP 6087 , AP 6088, DC 8040 Fluid, DC 8822A Fluid, DC 8803 & 8813 Polymers, 7-6030, AP-8104,AP 8201;
   Emulsionen: CE-8170 AF Microemulsion, 2-8177, 2-8194 Microemulsion, 9224 Emulsion, 939, 949, 959,DC 5-7113 Quat Microemulsion,DC 5-7070 Emulsion, DC CE-8810, CE 8401Emulsion, CE-1619, Dow Corning Toray SS-3551, Dow Corning Toray SS-3552;
*angeboten von der Gesellschaft Wacker:*
   Wacker Belsil ADM 652, ADM 656, 1100, 1600, 1650 (Fluide); ADM 6060 (lineare Amodimethicone) Emulsion; ADM 6057 E (verzweigt, Amodimethicone) Emulsion; ADM 8020 VP (Micro emulsion); SLM 28040 (Micro emulsion);
*angeboten von der Gesellschaft Momentive:*
   Silsoft 331, SF1708, SME 253 & 254 (Emulsion), SM2125 (Emulsion), SM 2658 (Emulsion), Silsoft Q (Emulsion);
*angeboten von der Gesellschaft Shin-Etsu:*
   KF-889, KF-867S, KF-8004, X-52-2265 (Emulsion);
*angeboten von der Gesellschaft Siltech Silicones:*
   Siltech E-2145, Siltech E-2145-35;
*angeboten von der Gesellschaft Evonik Industries:*
   Abil T Quat 60.

Besonders bevorzugt ist es, wenn die Silikonkombinationen **AP-8087;** Bis-Hydroxy/Methoxy Amodimethicone, Viscosity: 5000cP, Amine content: 0.5mol % diamine oder **DC 2-8566:** Amodimethicone; Viscosity: 3500cSt, Amine Content: 2.3 % mole amine % eingesetzt werden. Weitere Erfindungsgemäße Haarpflege Beispielsformulierungen sind hier abgebildet: Alle Konzentrationsangaben verstehen sich als Gewichtsanteile in %.

**Haarspülung:**

| | | **7** | **8** | **9** | **10** | **11** | **12** | **13** | **14** | **15** |
|---|---|---|---|---|---|---|---|---|---|---|
| 1 | Methylparaben | 0,25 | 0,25 | 0,25 | 0,25 | 0,25 | 0,25 | 0,25 | 0,25 | 0,25 |
| 2 | Propylparaben | 0,1 | 0,1 | 0,1 | 0,1 | 0,1 | 0,1 | 0,1 | 0,1 | 0,1 |
| 3 | Aqua | Ad.100 | Ad.100 | Ad. 100 | Ad.100 | Ad.100 | Ad.100 | Ad.100 | Ad. 100 | Ad.100 |
| 4 | Citric Acid | | 0,01 | 0,01 | | | | | | |
| 5 | Cetearyl Alcohol | | 3,5 | 4,44 | | | | | | |
| 6 | Cetyl Alcohol | 2,5 | | | 2,5 | 2,5 | 2,5 | 2 | 2,5 | 2,5 |
| 7 | Lactic Acid | 0,76 | | | 0,76 | 0,78 | 0,76 | 0,76 | 0,73 | 0,75 |
| 8 | Glycerin | | | 5 | | | | | | |
| 9 | Sodium Hydroxide | | 0,03 | 0,03 | | | | | | |
| 10 | Sodium Chloride | 0,01 | | | 0,01 | 0,01 | 0,01 | 0,01 | 0,01 | 0,01 |
| 11 | Stearyl Alcohol | 4,8 | | | 4,8 | 4,8 | 4,8 | 4 | 4,8 | 4,8 |
| 12 | Cetrimonium Chloride | | 2 | | | | | | | |
| 13 | Distearoylethyl Hydroxy-ethylmonium Methosulfate | | | 2,86 | | | | | | |
| 14 | Oryzanol | 0,05 | | 0,05 | 0,05 | 0,05 | 0,05 | 0,05 | 0,05 | 0,05 |
| 15 | Palmitamidopropyltrimonium Chloride | | | 1,66 | | | | 0,415 | | |
| 16 | Coco Betaine | 0,85 | | | 0,85 | 0,85 | 0,85 | 0,85 | 0,85 | 0,85 |
| 17 | Stearamidopropyl Dimethylamine | 2 | | | 1,5 | 2 | 2,2 | 2 | 2,2 | 2 |
| 18 | Silicone Quaternium-16 + Undeceth-11 + Butyloctanol + Undeceth-5 | 5 | 5 | 5 | 5 | 5 | | | 7,5 | |
| 19 | Silicone Quaternium-22 + PPG-3 Myristyl Ether | | | | | | | | | 7,7 |
| 20 | Behentrimonium Methosulfate | | 2,73 | | | | | | | |
| 21 | Dimethicone | | | | 1,54 | 3,08 | | | 2,31 | 2,5 |
| 22 | PPG-3 Benzyl Ether Myristate | | | | | | | 2 | | |
| 23 | Persea Gratissima (Avocado) Oil | 0,1 | | | | | | | | |
| 24 | Simmondsia Chinensis (Jojoba)Oil | | | | | | 0,1 | | | |
| 25 | Amodimethicone | | | 0,5 | | | 1 | 1 | | |
| 26 | Bis-Hydroxy/Methoxy Amodimethicone | | | | | | 0,5 | 0,5 | | |
| 27 | Parfum | 0,7 | 0,7 | 0,7 | 0,7 | 0,7 | 0,7 | 0,7 | 0,7 | 0,7 |
| 28 | Sodium Metabisulfite | 0,15 | 0,15 | 0,2 | 0,15, | 0,1 | 0,15 | 0,2 | 0,2 | 0,1 |
| 29 | Etidronic Acid | 0,25 | 0,18 | 0,3 | 0,3 | 0,18 | 0,3 | 0,3 | 0,4 | 0,3 |

| | **Tabelle 4.** | | |
|---|---|---|---|
| | **Handelsnamen der Rohstoffe (% aktiv Hauptkomponente):** | | |
| 1 | Nipagin M, Clariant, 99.5% | | |
| 2 | Nipasol M, Clariant, 99.5% | | |
| 4 | DSM Nutritional Products Europe, 100% | | |
| 5 | Nafol 1618-JA Sasol, 100% | | |
| 6 | Nacol 16-95, Sasol , 95% | | |
| 7 | 100366 Milchsäure 90, reinst DAB, Merck, 90% | | |
| 8 | Glycerol EP, vegetable, Spiga Nord, 99.7% | | |
| 11 | Lanette 18, Cognis , 100% | | |
| 12 | CTAC 6025 KC, KCl, 25% | | |
| 13 | Dehyquart F 75, Cognis, 69.935% | | |
| 14 | Gamma -Oryzanol, Biesterfeld, 98% | | |
| 15 | Varisoft PATC, Evonik Goldschmidt, 60% | | |
| 16 | Dehyton AB 30, Cognis, 31% | | |
| 17 | Tego Amid S18, Evonik Goldschmidt, 100% | | |
| 18 | DC 5-7113, Dow Corning, 22% | | |
| 19 | Abil T Quat 60, Evonik, 65%silicone | | |
| 20 | Incroquat Behenyl TMS-50, Croda, 55% | | |
| 21 | DC 5-7139 emulsion, 65% | | |
| 22 | Crodamol STS, Croda, 100% | | |
| 23 | Avocadoöl raffiniert, Gustav Heess, 100% | | |
| 24 | Jojobaöl Goldgelb, Gustav Heess, 100% | | |
| 25 | DC 2-8566, Dow Corning, 100% | | |
| 26 | AP-8087, Dow Corning, 100% | | |
| 28 | Natriumdisulfit food grade (E223), Biesterfeld | | |
| 29 | Turpinal SL, Thermphos Trading, 60% | | |

**Comb-In Cream:**

| | | **14** | **15** | **16** |
|---|---|---|---|---|
| 1 | Amodimethicone + Trideceth-12 + Cetrimonium Chloride | | 1,5 | |
| 2 | Methylparaben | 0,25 | 0,25 | 0,25 |
| 3 | Propylparaben | 0,1 | 0,1 | 0,1 |
| 4 | Aqua | Ad.100 | Ad. 100 | Ad.100 |
| 5 | Citric Acid | 0,01 | 0,01 | 0,01 |
| 6 | Cetearyl Alcohol | 3,5 | 3,0 | 3,5 |
| 7 | Glycerin | 9,0 | 5,0 | 9,0 |
| 8 | Sodium Hydroxide | 0,025 | 0,025 | 0,025 |
| 9 | Hydroxypropyl Methylcellulose | 0,25 | | 0,25 |
| 10 | Cetearyl Alcohol + Behentrimonium Chloride | | 1,0 | |
| 11 | Cetyl Alcohol | | 1,5 | |
| 12 | Cetrimonium Chloride | | 0,5 | |
| 13 | Distearoylethyl Hydroxyethylmonium Methosulfate + Cetearyl Alcohol | 2,5 | | 2,50 |
| 14 | Oryzanol | 0,01 | 0,01 | 0,01 |
| 15 | Cyclomethicone | 1,0 | | 1,0 |
| 16 | Palmitamidopropyltrimonium Chloride + Propylene Glycol | 1,0 | | 1,0 |
| 17 | Dimethicone + Cocamidopropyl Betaine + C12-15 Pareth-3 + Guar Hydroxypropyltrimonium Chloride | 0,5 | | |
| 18 | Amodimethicone | 1,5 | | 1,0 |
| 19 | Benzophenone-4 | | 0,25 | |
| 20 | Persea Gratissima (Avocado) Oil | 0,1 | | |
| 21 | Simmondsia Chinensis (Jojoba)Oil | | 0,1 | |
| 22 | Parfum | 0,7 | 0,7 | 0,7 |
| 23 | Sodium Metabisulfite | 0,15 | 0,15 | 0,15 |
| 24 | Etidronic Acid | 0,18 | 0,18 | 0,18 |

**Tabelle 5:**

| | **Handelsnamen der Rohstoffe (% aktiv Hauptkomponente):** |
|---|---|
| 1 | Dow Corning 959 Cationic Emulsion, 50% |
| 2 | Nipagin M, Clariant, 99.5% |
| 3 | Nipasol M, Clariant, 99.5% |
| 5 | DSM Nutritional Products Europe, 100% |
| 6 | Nafol 1618-JA Sasol, 100% |
| 7 | Glycerol EP, vegetable, Spiga Nord, 99.7% |
| 9 | Tylose E 707002, SE Tylose, 100% |
| 10 | Incroquat Behenyl TMC-25, HA 42212, Croda, 25% |
| 11 | Nacol 16-95, Sasol , 95% |
| 12 | CTAC 6025 KC, KCl, 25% |
| 13 | Dehyquart F 75, Cognis, 69.935% |
| 14 | Gamma -Oryzanol, Biesterfeld, 98% |
| 15 | Dow Corning 245 Fluid, 100% |
| 16 | Varisoft PATC, Evonik Goldschmidt, 60% |
| 17 | DC 5-7139 emulsion, 65% |
| 18 | Wacker-Belsil ADM 8020 VP, 15% |
| 19 | Uvasorb S 5, 3 V Sigma, 100% |
| 20 | Avocadoöl raffiniert, Gustav Heess, 100% |
| 21 | Jojobaöl Goldgelb, Gustav Heess, 100% |
| 23 | Natriumdisulfit food grade (E223), Biesterfeld |
| 24 | Turpinal SL, Thermphos Trading, 60% |

**Kur:**

| | | **14** | **15** |
|---|---|---|---|
| 1 | Amodimethicone + Trideceth-12 + Cetrimonium Chloride | 2,0 | |
| 2 | Methylparaben | 0,25 | 0,25 |
| 3 | Propylparaben | 0,1 | 0,1 |
| 4 | Aqua | Ad.100 | Ad.100 |
| 5 | Cetyl Alcohol | 3,5 | 2,9 |
| 6 | Stearyl Alcohol | 6,0 | 5,0 |
| 7 | Coco-Betaine | 0,85 | 0,85 |
| 8 | Cyclomethicone | 1,5 | |
| 9 | Cyclomethicone + Dimethiconol | | 2,0 |
| 10 | Bis-Hydroxy/Methoxy Amodimethicone | 1,0 | 1,0 |
| 11 | PPG-3 Benzyl Ether Myristate | 2,0 | |
| 12 | Stearamidopropyl Dimethylamine | 1,5 | 2,0 |
| 13 | Lactic Acid | 0,77 | 0,80 |
| 14 | Sodium Chloride | 0,01 | 0,01 |
| 15 | Oryzanol | 0,05 | 0,05 |
| 16 | Persea Gratissima (Avocado) Oil | 0,1 | |
| 17 | Simmondsia Chinensis (Jojoba)Oil | | 0,1 |
| 18 | Parfum | 0,7 | 0,7 |
| 19 | Sodium Metabisulfite | 0,15 | 0,15 |
| 20 | Etidronic Acid | 0,18 | 0,18 |

**Tabelle 6:**

| | **Handelsnamen der Rohstoffe (% aktiv Hauptkomponente):** |
|---|---|
| 1 | Dow Corning 959 Cationic Emulsion, 50% |
| 2 | Nipagin M, Clariant, 99.5% |
| 3 | Nipasol M, Clariant, 99.5% |
| 5 | Nacol 16-95, Sasol , 95% |
| 6 | Lanette 18, Cognis , 100% |
| 7 | Dehyton AB 30, Cognis, 31% |
| 8 | Dow Corning 245 Fluid, 100% |
| 10 | AP-8087, Dow Corning, 100% |
| 11 | Crodamol STS, Croda, 100% |
| 12 | Tego Amid S18, Evonik Goldschmidt, 100% |
| 13 | 100366 Milchsäure 90, reinst DAB, Merck, 90% |
| 15 | Gamma -Oryzanol, Biesterfeld, 98% |
| 16 | Avocadoöl raffiniert, Gustav Heess, 100% |
| 17 | Jojobaöl Goldgelb, Gustav Heess, 100% |
| 19 | Natriumdisulfit food grade (E223), Biesterfeld |
| 20 | Turpinal SL, Thermphos Trading, 60% |

## Patentansprüche

1. Haarkosmetische Zubereitung enthaltend Aminosilikone, Natriumdisulfit und Etidronsäure und oder deren Natrium- oder Kaliumsalz.

2. Zubereitung nach Anspruch 1 **dadurch gekennzeichnet, dass** höchstens 0,1% Farbstoffe enthält.

3. Zubereitung nach einem der vorangehenden Patentansprüche **dadurch gekennzeichnet, dass** ein kationisches Tensid enthalten ist.

4. Zubereitung nach einem der vorangehenden Patentansprüche **dadurch gekennzeichnet, dass** eine oder mehrere Fettverbindungen mit einem Schmelzpunkt oberhalb von 40 °C enthalten ist.

5. Zubereitung nach einem der vorangehenden Patentansprüche **dadurch gekennzeichnet, dass** das Aminosilikon 0.5 -2.5 mol% Aminogruppen enthält.

6. Zubereitung nach einem der vorangehenden Patentansprüche **dadurch gekennzeichnet, dass** sie eine Haarspülung ist.

7. Zubereitung nach einem der vorangehenden Patentansprüche **dadurch gekennzeichnet, dass** zwei verschiedene Aminosilikone enthalten sind.

8. Zubereitung nach Anspruch 7 **dadurch gekennzeichnet, dass** das zweite Aminosilikon als Fluid mit einer Viskosität von 1000cst bis 6000cst, vorzugsweise von 1000cst bis 5000cst vorliegt.

9. Zubereitung nach Anspruch 7 **dadurch gekennzeichnet, dass** das zweite Aminosilikon als Microemulsion von einem kationischen amino-functionalisertien Silikonpolymer mit einer Viskosität von 10,000cs vorliegt.

10. Zubereitung nach einem der vorangehenden Patentansprüche **dadurch gekennzeichnet, dass** als Aminosilikone Amino-funktionalisiertes Bis-Hydroxy/Methoxy Amodimethicone der Formel 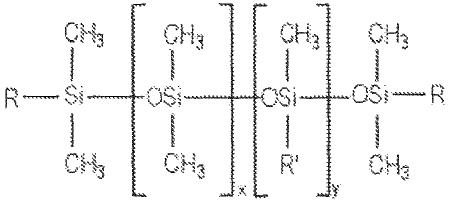 where R represents -OH or -OCH₃ and R' is 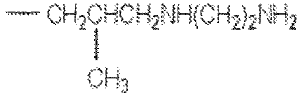 mit einer Viskosität von 5000cP und einem Amingehalt von 0.5mol% diamine oder Amodimethicone eingesetzt werden.

11. Zubereitung nach einem der vorangehenden Patentansprüche **dadurch gekennzeichnet, dass** 0.10-0.5 Gew.% besonders bevorzugt 0.15 bis 0.2 Gew. % Na-disulfit jeweils bezogen auf den Aktivgehalt enthalten sind.

12. Zubereitung nach einem der vorangehenden Patentansprüche **dadurch gekennzeichnet, dass** der Gehalt an Etidronsäure wenigstens dem Gehalt an Na-disulfit entspricht.

13. Zubereitung nach einem der vorangehenden Patentansprüche **dadurch gekennzeichnet, dass** der Gehalt an EDTA höchstens 0,01 Gew.% beträgt.

14. Zubereitung nach einem der vorangehenden Patentansprüche **dadurch gekennzeichnet, dass** der Gehalt an pflanzlichen Ölen gewählt aus der Gruppe Sonnenblumenöl, Babassuöl, Jojobaöl, Avocadoöl höchstens 1 Gew.% beträgt.

15. Zubereitung nach einem der vorangehenden Patentansprüche **dadurch gekennzeichnet, dass** der Gehalt an pflanzlichen Extrakten gewählt aus der Gruppe hydrolysierte Seide, Aloe, Kamille, lily of the valley höchstens 1 Gew.% beträgt.

16. Zubereitung nach einem der Patentansprüche 14 und 15 **dadurch gekennzeichnet, dass** der Gehalt an pflanzlichen Ölen und an pflanzlichen Extrakten zusammengenommen höchstens 1 Gew.% beträgt.

17. Zubereitung nach einem der vorangehenden Patentansprüche **dadurch gekennzeichnet, dass** der Gehalt an pflanzlichen Ölen höchstens 1 Gew.% beträgt.

18. Verwendung von Natriumdisulfit und Etidronsäure zur Verhinderung des Vergilbens haarkosmetischer Zubereitungen mit Aminosilikonen.

## Claims

1. Cosmetic hair preparation comprising aminosilicones, sodium disulfite and etidronic acid and or the sodium or potassium salt thereof.

2. Preparation according to Claim 1, **characterized in that** said preparation comprises at most 0.1% dye.

3. Preparation according to either of the preceding patent claims, **characterized in that** a cationic surfactant is present.

4. Preparation according to any of the preceding patent claims, **characterized in that** one or more fatty compounds are present having a melting point above 40°C.

5. Preparation according to any of the preceding patent claims, **characterized in that** the aminosilicone comprises 0.5-2.5 mol% amino groups.

6. Preparation according to any of the preceding patent claims, **characterized in that** said preparation is a hair conditioner.

7. Preparation according to any of the preceding patent claims, **characterized in that** two different aminosilicones are present.

8. Preparation according to Claim 7, **characterized in that** the second aminosilicone is present as a fluid having a viscosity of 1000 cSt to 6000 cSt, preferably 1000 cSt to 5000 cSt.

9. Preparation according to Claim 7, **characterized in that** the second aminosilicone is present as a microemulsion of a cationic amino-functionalized silicone polymer having a viscosity of 10 000 cSt.

10. Preparation according to any of the preceding patent claims, **characterized in that** the aminosilicones used are amino-functionalized bis-hydroxy/methoxy amodimethicones of the formula 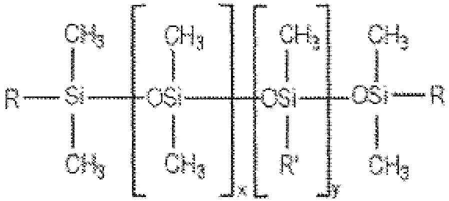 where R represents -OH or -OCH₃ and R' is 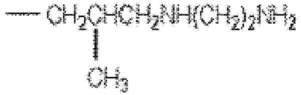 having a viscosity of 5000 cP and an amine content of 0.5 mol% diamines or amodimethicones.

11. Preparation according to any of the preceding patent claims, **characterized in that** 0.10-0.5 wt%, particularly preferably 0.15 to 0.2 wt% Na disulfite is present, in each case based on the active content.

12. Preparation according to any of the preceding patent claims, **characterized in that** the etidronic acid content corresponds at least to the Na disulfite content.

13. Preparation according to any of the preceding patent claims, **characterized in that** the EDTA content is at most 0.01 wt%.

14. Preparation according to any of the preceding patent claims, **characterized in that** the content of vegetable oils selected from the group of sunflower oil, babassu oil, jojoba oil, avocado oil is at most 1 wt%.

15. Preparation according to any of the preceding patent claims, **characterized in that** the content of plant extracts selected from the group of hydrolysed silk, aloe, camomile, lily of the valley is at most 1 wt%.

16. Preparation according to either of patent claims 14 and 15, **characterized in that** the combined content of vegetable oils and plant extracts is at most 1 wt%.

17. Preparation according to any of the preceding patent claims, **characterized in that** the content of vegetable oils is at most 1 wt%.

18. Use of sodium disulfite and etidronic acid for preventing yellowing of cosmetic hair preparations with aminosilicones.

## Revendications

1. Préparation cosmétique capillaire contenant des aminosilicones, du disulfite de sodium et de l'acide étidronique et/ou son sel de sodium ou de potassium.

2. Préparation selon la revendication 1, **caractérisée en ce qu'**elle contient au plus 0,1 % de colorants.

3. Préparation selon l'une quelconque des revendications précédentes, **caractérisée en ce qu'**un tensioactif cationique est contenu.

4. Préparation selon l'une quelconque des revendications précédentes, **caractérisée en ce qu'**un ou plusieurs composés gras ayant un point de fusion supérieur à 40 °C sont contenus.

5. Préparation selon l'une quelconque des revendications précédentes, **caractérisée en ce que** l'aminosilicone contient 0,5 à 2,5 % en moles de groupes amino.

6. Préparation selon l'une quelconque des revendications précédentes, **caractérisée en ce qu'**il s'agit d'un après-shampoing.

7. Préparation selon l'une quelconque des revendications précédentes, **caractérisée en ce que** deux aminosilicones différentes sont contenues.

8. Préparation selon la revendication 7, **caractérisée en ce que** la seconde aminosilicone se présente sous la forme d'un fluide ayant une viscosité de 1 000 cst à 6 000 cst, de préférence de 1 000 cst à 5 000 cst.

9. Préparation selon la revendication 7, **caractérisée en ce que** la seconde aminosilicone se présente sous la forme d'une microémulsion d'un polymère de silicone cationique à fonctionnalisation amino ayant une viscosité de 10 000 cs.

10. Préparation selon l'une quelconque des revendications précédentes, **caractérisée en ce qu'**en tant qu'aminosilicones, des bis-hydroxy/méthoxy-amodiméthicones à fonctionnalisation amino de formule 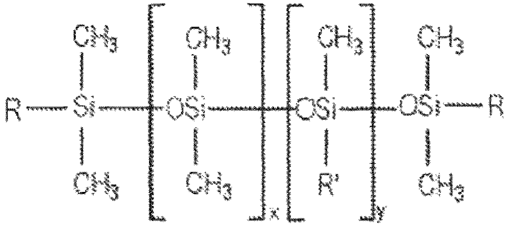 dans laquelle R représente -OH ou -OCH₃ et R' représente 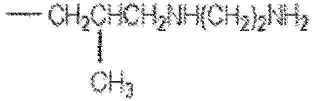 ayant une viscosité de 5 000 cP et une teneur en amines de 0,5 % en moles de diamines ou d'amodiméthicones, sont utilisées.

11. Préparation selon l'une quelconque des revendications précédentes, **caractérisée en ce que** 0,10 à 0,5 % en poids, de manière particulièrement préférée 0,15 à 0,2 % en poids, de disulfite de Na est contenu, à chaque fois par rapport à la teneur en agents actifs.

12. Préparation selon l'une quelconque des revendications précédentes, **caractérisée en ce que** la teneur en acide étidronique correspond au moins à la teneur en disulfite de Na.

13. Préparation selon l'une quelconque des revendications précédentes, **caractérisée en ce que** la teneur en EDTA est d'au plus 0,01 % en poids.

14. Préparation selon l'une quelconque des revendications précédentes, **caractérisée en ce que** la teneur en huiles végétales choisies dans le groupe constitué par l'huile de tournesol, l'huile de babassu, l'huile de jojoba, l'huile d'avocat est d'au plus 1 % en poids.

15. Préparation selon l'une quelconque des revendications précédentes, **caractérisée en ce que** la teneur en extraits végétaux choisis dans le groupe constitué par la soie hydrolysée, l'aloès, la camomille, le muguet est d'au plus 1 % en poids.

16. Préparation selon l'une quelconque des revendications 14 et 15, **caractérisée en ce que** la teneur en huiles végétales et en extraits végétaux pris ensemble est d'au plus 1 % en poids.

17. Préparation selon l'une quelconque des revendications précédentes, **caractérisée en ce que** la teneur en huiles végétales est d'au plus 1 % en poids.

18. Utilisation de disulfite de sodium et d'acide étidronique pour empêcher le jaunissement de préparations cosmétiques capillaires contenant des aminosilicones.
